# EUROPÄISCHE PATENTSCHRIFT

(11) **EP 1 610 269 B1**
(45) Veröffentlichungstag und Bekanntmachung des Hinweises auf die Patenterteilung: **28.03.2007**
(21) Anmeldenummer: 05107181.9
(22) Anmeldetag: 21.02.2002
(51) Int. Cl.: G06T 7/00, G06T 5/00, A61B 6/14, G06Q 50/00, G06F 17/30

(54) **Verfahren und Anordnung zur Zuordnung von Informationen zu digitalen Röntgenaufnahmen**
Method and apparatus for associating information to digital radiographs
Procédé et appareil d'association des informations aux radiographies numériques

(30) Priorität: 21.02.2001 DE 10108295
(43) Veröffentlichungstag der Anmeldung: 28.12.2005
(62) Teilanmeldung aus: 02716606.5
(73) Patentinhaber: Sirona Dental Systems GmbH, 64625 Bensheim (DE)
(72) Erfinder: Zimmermann, Jürgen, 64585, Biebesheim (DE)
(74) Vertreter: Sommer, Peter

(56) Entgegenhaltungen:
- EP-A- 0 487 110
- EP-A- 0 757 309
- EP-A- 0 973 116
- WO-A-01/46895

## Beschreibung

Die Erfindung betrifft und eine Anordnung zur Zuordnung von Informationen zu Objekten, genauer Zähnen, die in einer digitalisierte Röntgenaufnahme oder einer schematischen Darstellung bestimmt sind.

Ein Röntgengerät zur Erstellung von Panorama-Schichtaufnahmen und Einzelaufnahmen hiervon ist aus der DE 35 45 509 (US 4,847,881) und der DE 35 45 493 (US 4,813,060) bekannt. Digitale Röntgenaufnahmen für Panoramaschichtaufnahmen und cephalometrische Aufnahmen sind aus der EP 0 632 994 (US 5,511,106) bekannt. Die Erstellung digitaler Intraoral-Aufnahmen mit einem Intraoralsensor sind aus der EP 0 643 901 (US 5,513,252) bekannt.

Viele Verfahren der Diagnostik beziehen sich auf einzelne Details wie z.B. einzelnen Zähne, deren Existenz, Form, Lage individuell verschieden ist. Die Diagnostik und Dokumentation wird dadurch erschwert, dass die Anwender gezwungen sind, bei weiteren Arbeiten auf nicht-individuelle, sondern allgemeine Schemata auszuweichen wie z.B. das feste Zahnschema auf dem Krankenschein.

Bei automatisierten Aufnahmeserien ist es notwendig, die zu treffenden Zähne vorher im Bedienprogramm zu spezifizieren. Dies ist momentan entweder unzureichend oder umständlich gelöst, da es entweder nur möglich ist, ganze, fix festgelegte Zahngruppen auszuwählen, oder nur einzelnen Zähne zuzuordnen. In jedem Fall geht der reale Bezug verloren, da der Bediener die vertraute Umgebung, z.B. eine reale Gebissaufnahme, verlassen muss.

Die EP 0 973 116 A1 offenbart ein Verarbeitungssystem für medizinische Informationen zur Unterstützung der Diagnose, welches Speichermittel zur Speicherung von medizinischen Bildern, Eingabemittel zur Eingabe von neuen medizinische Bildern, Erkennungsmittel zur Erfassung von Abnormalitäten in den neu eingegebenen medizinischen Bildern, Auswahlmittel zur Auswahl eines medizinischen Bildes im Zusammenhang mit der Abnormalität, die in dem neu eingegebenen Bild erkannt wird und Anzeigemittel zur gleichzeitigen Anzeige des ausgewählten und des untersuchten Bildes. Weiterhin ist es möglich, Informationen zu Objekten zuzuordnen.

Die EP 0 487 110 A2 offenbart ein computerunterstütztes Diagnosesystem für medizinische Verwendung. Verschiedene Objekte können hierarchisch miteinander verknüpft werden.

Panorama-Röntgenaufnahmen dienen dazu, schnell eine Übersicht über den Gesamtzustand des Gebisses zu erhalten. Hiervon lassen sich bereits Befunde ohne weitere, z.B. intraorale, Röntgenaufnahmen ableiten. Diese Befunde beziehen sich aber nicht auf das gesamte Bild, sondern jeweils auf ein bestimmtes dargestelltes Objekt, z.B. einen bestimmten Zahn. Damit entstehen letztlich mehrere Befunde, die sich auf ein Bild beziehen und die z.B. den einzelnen Zähnen zugeordnet sind. Deren Erfassung ist umständlich, da der Anwender i.d.R. die unmittelbare Bedienumgebung des Panoramabildes verlassen muss.

Aufgabe der vorliegenden Erfindung ist es, ein individuelles Zahnschema bereitzustellen und diesem Informationen zuzuordnen.

### Darstellung der Erfindung

Gelöst wird diese Aufgabe durch Anordnungen, die die Merkmale des unabhängigen Anspruchs aufweisen.

Ein Bestandteil der vorliegenden Erfindung ist eine Anordnung, die erkannten Objekten Informationen zuordnet. Dieser Anordnung kann auf den bereits oben beschriebenen Anordnungen bzw. Verfahren aufbauen. Die Anordnung kann jedoch auch separat von ihnen verwendet werden, wenn bereits Objekte vorliegen. Es handelt sich hierbei um eine Anordnung zur Zuordnung von Informationen zu Objekten. Bei diesen Objekten handelt es sich um Zähne, die in digitalisierte Röntgenaufnahme bestimmt sind.

Die Anordnung weist Ein- und Ausgabegeräte zur interaktiven Steuerung der Anordnung auf. Bei diesen Geräten handelt es sich vorzugsweise um Tastaturen, Zeigegeräte und Bildschirme. In einem ersten Speicherbereich sind Röntgenaufnahme abgelegt. Hierbei handelt es sich vorzugsweise um einen Containern für eine Vielzahl von digitalisierten Röntgenaufnahmen. Zusätzlich zu diesen Röntgenaufnahmen sind Informationen abgelegt, die die Objekte in der Röntgenaufnahme kennzeichnen. Die Objektkennzeichnungsinformationen haben die Aufgabe, den flächigen oder gegebenenfalls auch den räumlichen Umfang des Objektes in der Darstellung zu bestimmen. Es handelt sich vorzugsweise um separate Datenstrukturen, die durch zwei- oder mehrdimensionale Polygone das Objekt in der Röntgenaufnahme darstellen. Diese Informationen werden beim Bildaufbau über die ursprüngliche Röntgenaufnahme gelegt. Es ist jedoch auch denkbar, dass diese Informationen in die Röntgenaufnahme selber integriert werden.

In einem zweiten Speicherbereich werden die Informationen zu den Objekten abgelegt. Bei diesen Informationen kann es sich um Textinformationen oder auch um Bildinformationen handeln. So ist denkbar, dass eine Detailaufnahme in höherer Auflösung als zusätzliche Informationen gespeichert wird. Eine andere Möglichkeit besteht darin, durch grafische Markierungen einen Hinweis darauf zugeben, in welchen Bereichen Behandlungen notwendig sind oder bereits vorgenommen wurden. Es ist ebenfalls möglich mehrere Arten von Informationen einen Objekt zuzuordnen.

Weiterhin werden Referenzen zwischen den Objekten und den Informationen gespeichert. Bei diesen Referenzen handelt es sich vorzugsweise um Pointer bzw. Index-Felder, die die logische Verbindungen zwischen den Objekten und den Informationen herstellen.

Eine Bearbeitungseinheit kontrollierten die Operationen Anlegen, Löschen und Zugriff. Die Operationen werden vorzugsweise durch den Benutzer mit Hilfe der Ein- und Ausgabegeräte gestartet. Die Bearbeitungseinheit steuert somit den Zugriff auf die unterschiedlichen Speicherbereiche.

Bei diesen Speicherbereichen handelt es sich vorzugsweise nur um logisch unterschiedliche Speicherbereiche. Er kann sich physikalisch gesehen um einen zusammenhängenden Speicherbereich handeln.

Die Verwendung eines physikalischen und logischen Speicherbereichs für die Bildinformationen, die Objektinformationen und die zusätzliche Informationen ist möglich, jedoch nicht besonders vorteilhafte, da die Flexibilität verloren geht.

Um die Arbeit mit der Anordnung zu verbessern, sind die Objekte auf dem Ausgabegerät optisch hervorgehoben. Sollten auswählbare Informationen vorliegen, so werden diese durch ein weiteres Merkmal grafisch gekennzeichnet.

Die Verwendung von Referenzen ermöglicht eine hierarchische Anordnung von Informationen. Der Benutzer kann sich somit von einer Panoramaaufnahme zu Detailaufnahmen bewegen und Informationen auf jeder Ebene hinterlegen.

Vorteilhaft ist eine Anordnung, bei der die Referenzen in Form von Verweisen (Links) entweder unmittelbar beim Objekt und/oder unmittelbar bei der Information und/oder separat verwaltet werden.

In einer bevorzugten Ausgestaltung ist die Anordnung einen PC mit einem Bildschirme, wobei die Informationen durch KontextMenüs zugänglich sind. Dieser Kontextmenüs werden in Form von Pop-Up-Fenster dargestellt, wenn ein Objekt durch den Benutzer aktiviert wird. Über das Kontext-Menü kann der Benutzer entscheiden, ob er neue Informationen hinterlegen möchte, oder ob es sich bereits vorliegende Informationen anschauen möchte.

Die weiteren Informationen sind vorzugsweise Diagnose- und/oder Behandlungsinformationen und/oder weitere insbesondere detaillierte Röntgenaufnahmen.

In einer bevorzugten Ausführungsform weist die Anordnung eine Schnittstelle zu einem Röntgengerät auf. Über diese Schnittstelle werden vom Röntgengerät Informationen in Form von Röntgenaufnahmen an die Anordnung übertragen, die im ersten oder dritten Speicherbereich abgelegt werden. Hat der Benutzer bereits zu Beginn ein Objekt bestimmten, so kann ebenfalls sofort eine Referenz im vierten Speicherbereich zu diesem Objekt angelegt werden. Die neue Röntgenaufnahme wird sofort einer betehenden Röntgenaufnahme oder einem ausgewählten Teilbereich davon zugeordnet und mit einer entsprechenden Referenz versehen. Eine hierarchische Anordnung ist somit ohne weiteres möglich, die sich gegebenenfalls über mehrere Ebenen erstrecken kann.

Sollte ein Objekt nicht bereits bestimmt worden sein, so stellt die Anordnungsmittel bereit, die eine manuelle Auswahl eines bestimmten Bereichs auf der Röntgenaufnahme zulassen.

Die Anordnung weist vorzugsweise die Funktionalität eines Datenbanksystems auf.

Vorzugsweise werden die Objekte auf einer Anordnung mit einem Verfahren erkannt, wie es bereits oben beschrieben wurde. Eine Kombination der Anordnungen ist somit denkbar.

Bei einem Verfahren zur Zuordnung von Informationen zu Objekten, die Zähnen, die in einer digitalisierte Röntgenaufnahme bestimmt sind, wird in einem ersten Schritt das digitalisierte Röntgenbild dargestellt. In einem zweiten Schritt werden die Objekte manuell oder automatisch bestimmt. Dieser Schritt ist jedoch nur dann notwendig, wenn die Objekte nicht bereits schon bestimmt sind. In einem dritten Schritt wird das Objekt ausgewählt, für das weitere Informationen gespeichert, abgerufen oder gelöscht werden sollen.

Die wohl häufigste Operation ist die Abfrageoperationen, bei der Informationen aus der Datenbank geladen werden. Hierbei wird einer Referenz gefolgt, die in Relation zum Objekt abgelegt ist, um anhand dieser Referenz die Informationen zu bestimmten, die dargestellt werden sollen.

Bei einer Löschoperation wird ebenfalls der Referenz gefolgt wird, die in Relation zum Objekt abgelegt ist. Diese Referenz und ggfs. die Information werden daraufhin gelöscht. Eine Information wird immer nur dann gelöscht, wenn sie lediglich von einem Objekt referenziert wird. Es kann der Fall eintreten, dass mehrere Objekte eine Information referenzieren. Wird in diesem Falle ebenfalls die Informationen gelöscht würde, so fehlt für zumindest ein Objekt die Informationen.
Bei einer Speicheroperation wird ein Objekt ausgewählt und Speicherbereich für die Information bereitgestellt. Weiterhin wird ein Speicherbereich für die Referenz bereitgestellt, um dann die neuen Informationen und die entsprechend Referenz in diesen Speicherbereichen abzulegen.

Es ist ebenfalls denkbar, dass nach der Bestimmung des Objektes digitale Abbildungen vom Röntgengerät empfangen werden, die automatisch dem bestimmten Objekt zugeordnet werden. Hierbei wird ausreichend viel Speicherbereich bereitgestellt (allokiert), um die Informationen abzulegen.

In einer bevorzugten Ausgestaltung sind die Informationen grafische Markierungen, die als Overlay über die Aufnahmen gelegt werden können. Hierdurch ist es möglich Detailinformationen, die auf der Abbildungen nicht zuerkennen waren, nachträglich zu verfeinern und zu bearbeiten.

Für den Fall, dass Objekte in weitere Bereiche aufgeteilt werden sollten, stellt das Verfahren die Möglichkeit zur Verfügung, dass Bereiche der Objekte bestimmt werden können, in denen Informationen zugeordnet werden können.

Der Zugriff auf die Informationen erfolgt vorzugsweise durch Kontextmenüs, die zu den einzelnen Objekten abgerufen werden können.

Die Verfahren werden vorzugsweise durch Software realisiert, die auf einen bekannten PC abläuft.

Es folgt eine detaillierte Beschreibung anhand der Zeichnungen. Es zeigt:
- Fig. 1: den schematischen Ablauf bei der Bestimmung von Zähnen;
- Fig. 2: Informationen, die zu einem Objekt (Zahn) abgelegen wurden;
- Fig. 3: Panoramaaufnahme mit einem erkannten Zahn
- Fig. 4a, b: ein Kontextmenü für ein Objekt.

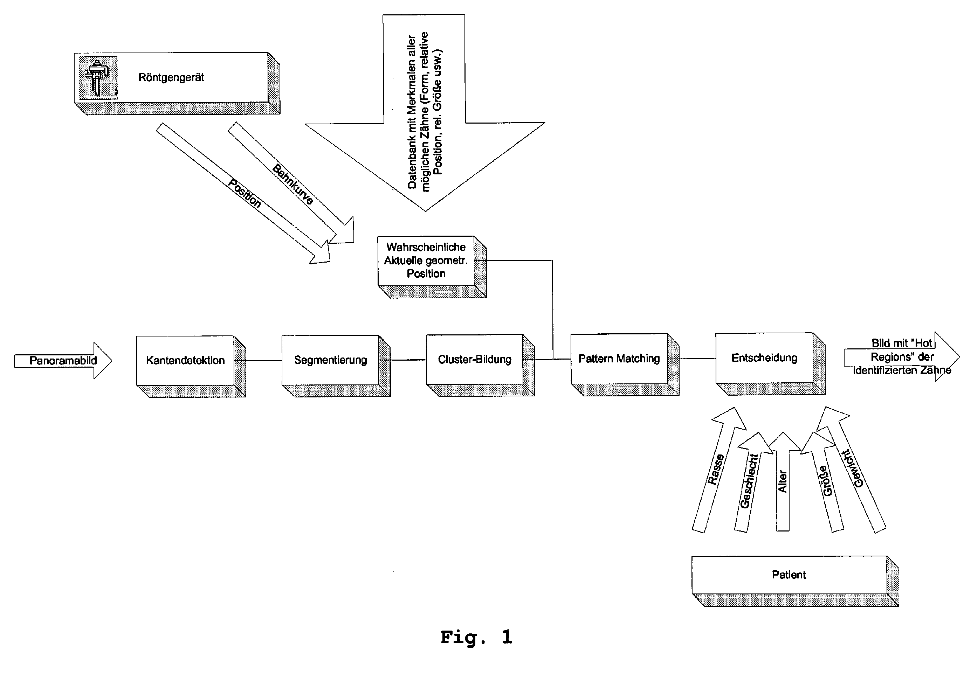

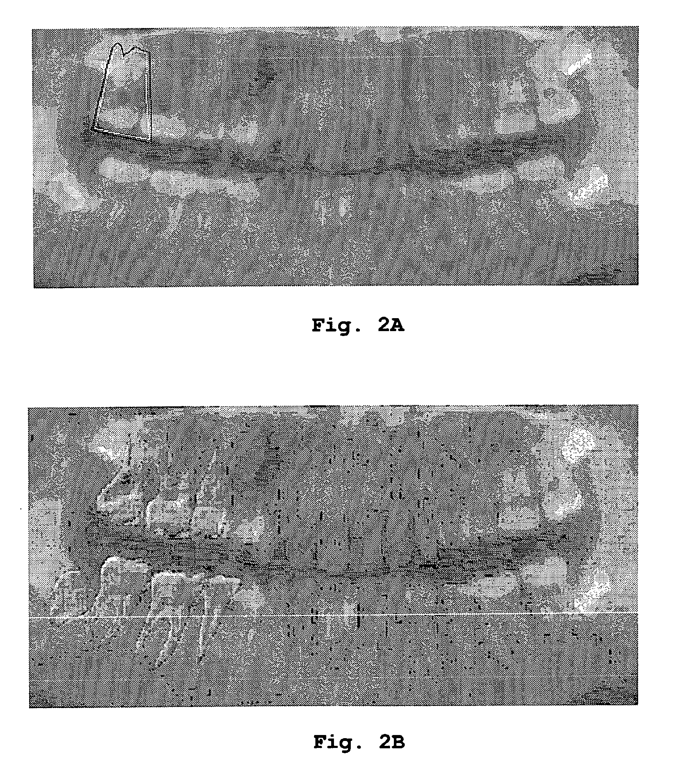

Die Fig. 1 zeigt schematisch, welche Informationen vom Röntgengerät und vom Patient nach der Bilderkennung durch Kantenfindung und Segmentierung verwendet werden können. Sollten Kanten und Segmente gefunden worden sein, so wird versucht, durch Cluster-Bildung die erkannten Bereiche und Kanten zu gruppieren. Um den entsprechenden Zahn zu identifizieren, werden unter Berücksichtigung der Geräteparameter diese Gruppen mit Zahnformen aus einer patientenunabhängigen Datenbank verglichen. Unter Berücksichtigung der patientenabhängigen Parameter wird dann eine weitere Verarbeitung durchgeführt. Als Ergebnis wird eine Röntgenaufnahme dargestellt, die durch entsprechende Regionen, d. h. Objekte, bestimmt und unterteilt ist. Diese Regionen stehen für die erkannten Zähnen. Eine solche Region ist den Fig. 2a, 2b zu entnehmen.

Die Fig. 2a zeigt einen erkannten Zahn, für den Informationen hinterlegt werden können. Der erkannte Zahn wird durch eine entsprechende Umrandung dargestellt.

In Fig. 2b ist auf der linken Bildhälfte eine Gruppe von Zähnen durch einen Mausklick ausgewählt worden und ist als Folge davon in ihrer Kontur hervorgehoben dargestellt. Es handelt sich um Zähne in der oberen und unteren Zahnreihe. Den so ausgewählten Zähnen können nun Informationen zugeordnet werden. Werden aber z.B. zu viele Zähne erfasst oder soll die Sensorpositionierung etwas verschoben werden oder sollen erfaßte Zähne keine Rolle spielen, da sie zwar zu sehen, jedoch nicht Gegenstand der Diagnose sein werden, kann eine An- bzw. Abwahl einzelner oder mehrerer Zähne durch z.B. übliche Mausaktionen wie Einfach- oder Doppelklick, Aufziehen eines Bereiches usw. direkt über den ausgewählten Bereichen des Hintergrundbildes erfolgen. Insbesondere für Aufnahmeserien ist es notwendig, die zu treffenden Zähne vorher im Bedienprogramm einzeln zu spezifizieren.

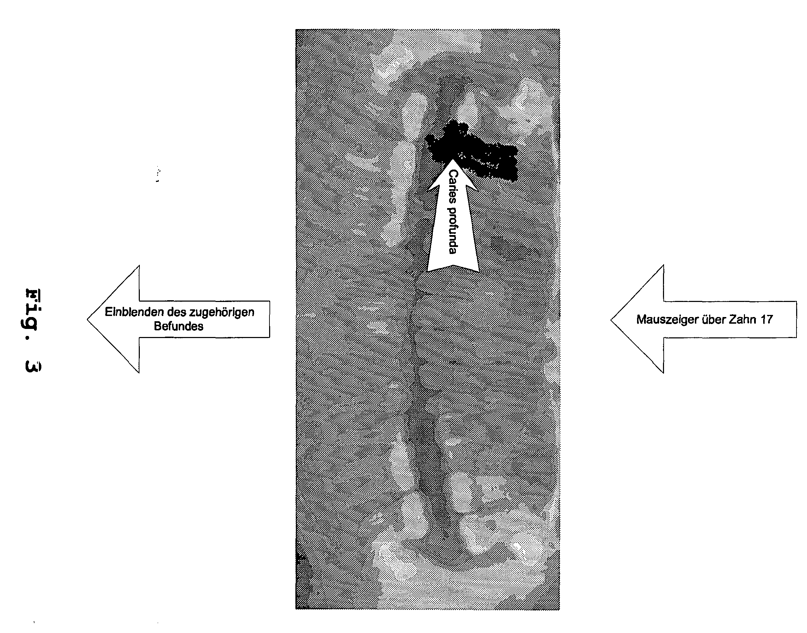

Fig. 3 zeigt eine mögliche Darstellungsform von Informationen, die Objekten zugeordnet wurden. Im vorliegenden Fall handelt es sich um Karies. Dieser Hinweis wird immer dann angezeigten, wenn der Mauszeiger über den entsprechenden Zahn gefahren wird. Andere Darstellungsformen sind jedoch auch denkbar, insbesondere mit kleinen grafischen Symbolen, die darauf hinweisen, dass weitere Informationen für dieses Objekt hinterlegt sind.

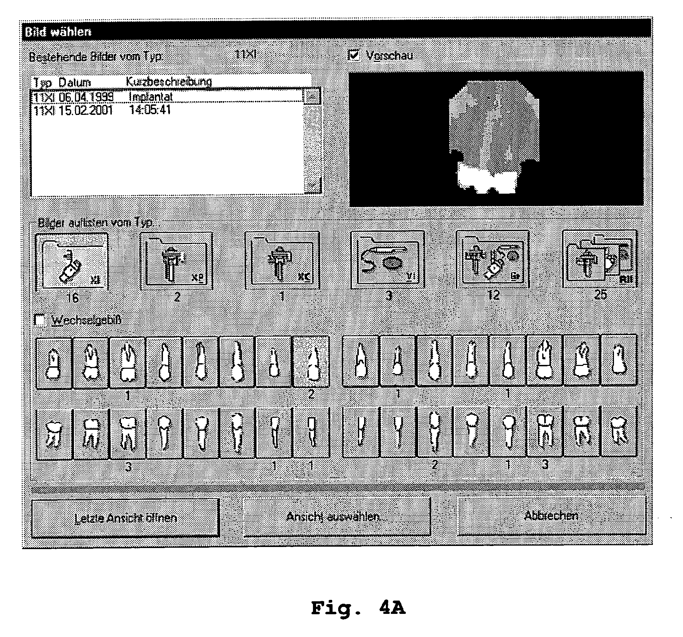

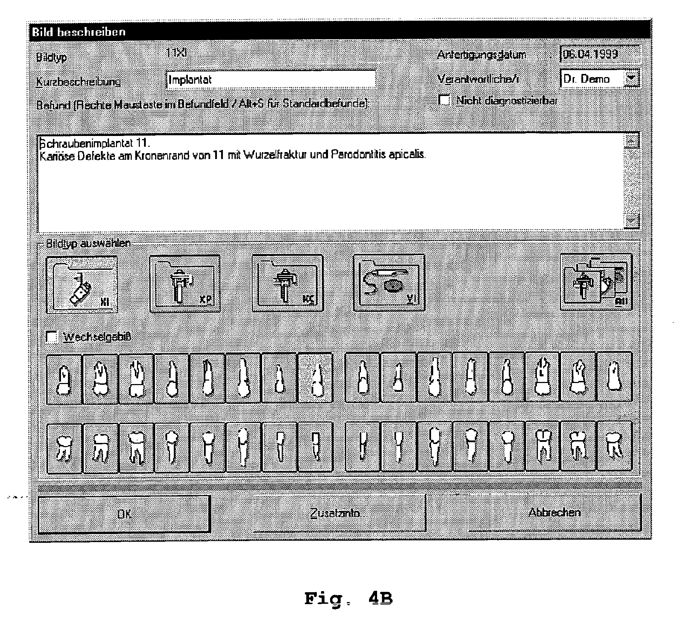

Die Figuren 4a und 4b zeigen eine mögliche Verwaltung von digitalen Röntgenaufnahmen. Hierbei wird jedem Zahn, so weit er vorhanden ist eine entsprechenden Röntgenaufnahme zugeordnet. Diese Zuordnung kann natürlich nur dann erfolgen, wenn entsprechende Aufnahmen vorliegen. Die Figuren 4a und 4b zeigen die Möglichkeit auf, dass die Objekte nicht auf einer Röntgenaufnahme abgegrenzt sein müssen, sondern auch auf einer schematischen Darstellung angeordnet sein können.

## Patentansprüche

1. Anordnung zur Zuordnung von Informationen zu Objekten, die in einer digitalisierte Röntgenaufnahme oder einer schematischen Darstellung bestimmt sind,
- mit einem Ein- und Ausgabegerät zur interaktiven Steuerung der Anordnung,
- mit einem Speicherbereich, in dem die Röntgenaufnahme oder die schematische Darstellung abgelegt ist, wobei den Röntgenaufnahmen oder den schematischen Darstellungen Objektkennzeichnungsinformationen zugeordnet sind,
- mit einem zweiten Speicherbereich, in dem Informationen zu den Objekten abgelegt sind, wobei Referenzen zwischen den Objekten und den Objektkennzeichnungsinformationen gespeichert werden,
- mit einer Bearbeitungseinheit, die Anlege-, Lösch- und/oder Zugriffsoperationen auf die Speicherbereiche kontrolliert und die Referenzen verwaltet, wobei die Operationen vorzugsweise durch das Eingabegerät angestoßen werden und auf dem Ausgabegerät dargestellt werden, **dadurch gekennzeichnet, dass** die Objekte Zähne sind, und dass die Objekte auf dem Ausgabegerät optisch hervorgehoben dargestellt und weiterhin auswählbar sind, um die hinterlegten Informationen abzufragen.

2. Anordnung nach Anspruch 1, **dadurch gekennzeichnet, dass** in Folge der Auswahl eines Objektes zu weiteren Informationen verzweigt wird, so weit sie vorhanden sind.

3. Anordnung nach Anspruch 1 oder 2, **gekennzeichnet durch** eine Schnittstelle zu einem Röntgengerät, wobei das Röntgengerät über die Schnittstelle die Informationen in Form von Daten zur Darstellung als Röntgenaufnahmen sendet, wobei diese Informationen in einem dritten Speicherbereich abgelegt werden und eine Referenz in einem vierten Speicherbereich zu einem Objekt angelegt wird.

4. Anordnung nach einem oder mehreren der Ansprüche 1 bis 3, **dadurch gekennzeichnet, dass** die Informationen über mehrere Ebenen hierarchisch angeordnet sein können.

5. Anordnung nach einem oder mehreren der Ansprüche 1 bis 4, **gekennzeichnet durch** Mittel, die es erlauben, dass die Objekte manuell **durch** Auswahl eines bestimmten Bereichs auf der Röntgenaufnahme bestimmt werden können.

6. Anordnung zur Identifikation von Zähnen **gekennzeichnet durch** die Merkmale einer Anordnung nach einem oder mehreren der vorhergehenden Ansprüche.

## Claims

1. A system for assigning information to objects, identified in a digitized X-ray image or diagrammatic representation, comprising:
- an input device and output device for interactive control of the system,
- a memory area, in which the X-ray image or diagrammatic representation is stored, items of object-labeling information being assigned to said X-ray image or diagrammatic representation,
- a second memory area, in which items of information relating to said objects are stored together with references linking said objects to said object-labeling information,
- a processing unit that controls the storing, deleting, and/or accessing operations for said memory areas and administers said reference links, which operations are preferably initiated by said input device and displayed on said output device,
**characterized in that** the objects are teeth and the objects are optically highlighted on the output device and can be selected in order to query the deposited information.

2. A system as defined in claim 1, **characterized in that** the selection of an object affords branching to further information, if present.

3. A system as defined in claim 1 or claim 2, **characterized by** a computer interface for an X-ray apparatus by means of which items of information are sent, via said computer interface, in the form of data intended to by displayed as an X-ray image, which items of information are stored in a third memory area and a reference link to an object is stored in a fourth memory area.

4. A system as defined in any one or more of claims 1 to 3, **characterized in that** said items of information can be arranged in a hierarchy over a plurality of levels.

5. A system as defined in any one or more of claims 1 to 4, **characterized by** means for allowing said objects to be manually defined by selecting a specific region of the X-ray image.

6. A system for the identification of teeth, **characterized by** the features of a system as defined in any one or more of the previous claims.

## Revendications

1. Dispositif pour l'association d'informations à des objets définis par radiographie numérique ou par représentation schématique
- avec un appareil d'entrée et de sortie pour la commande interactive du dispositif,
- avec une zone de mémoire où la radiographie ou la représentation schématique est archivée, les radiographies ou les représentations schématiques étant associées à des informations de caractérisation d'objet,
- avec une deuxième zone de mémoire où des informations sur les objets sont archivées, des références entre les objets et les informations de caractérisation d'objet étant mémorisées,
- avec une unité de traitement contrôlant des opérations de création, d'effacement et/ou d'accès dans les zones de mémoire et administrant les références, les opérations étant préférentiellement activées par l'appareil d'entrée et représentées sur l'appareil de sortie, **caractérisé en ce que** les objets sont des dents et **en ce que** les objets sont optiquement marqués sur l'appareil de sortie et en outre sélectionnables pour appeler les informations archivées.

2. Dispositif selon la revendication 1, **caractérisé en ce que**, suite à la sélection d'un objet, il est renvoyé vers d'autres informations si celles-ci existent.

3. Dispositif selon la revendication 1 ou 2, **caractérisé par** une interface vers un appareil radiographique, ledit appareil radiographique adressant les informations via l'interface, sous forme de données pour la représentation en tant que radiographies, ces informations étant archivées dans une troisième zone de mémoire et une référence à un objet étant créée dans une quatrième zone de mémoire.

4. Dispositif selon l'une ou plusieurs des revendications 1 à 3, **caractérisé en ce que** les informations peuvent être hiérarchiquement réparties sur plusieurs niveaux.

5. Dispositif selon l'une ou plusieurs des revendications 1 à 4, **caractérisé par** des moyens permettant de définir manuellement les objets par sélection d'une zone précise sur la radiographie.

6. Dispositif d'identification de dents, **caractérisé par** les caractéristiques d'un dispositif selon l'une ou plusieurs des revendications précédentes.
